Europäisches Patentamt

**European Patent Office**

**Office européen des brevets**

(11) Publication number: **0 125 424**
**A1**

## (12) EUROPEAN PATENT APPLICATION

(21) Application number: **84102860.8**

(22) Date of filing: **15.03.84**

(51) Int. Cl.³: **A 61 M 16/00**

(30) Priority: **15.03.83 ZA 831790**

(43) Date of publication of application:
**21.11.84 Bulletin 84/47**

(84) Designated Contracting States:
**AT BE CH DE FR GB IT LI LU NL SE**

(71) Applicant: **van den Bergh, Jozef Erasmus**
**4 Waterkloof**
**Rustenburg Transvaal Province(ZA)**

(72) Inventor: **van den Bergh, Jozef Erasmus**
**4 Waterkloof**
**Rustenburg Transvaal Province(ZA)**

(74) Representative: **Stapf, Otto F., Dipl.-Ing.**
**Patentanwälte Dr. Berg Dipl.-Ing. Stapf Dipl.-Ing.**
**Schwabe Dr. Dr. Sandmair Postfach 86 02 45**
**Stuntzstrasse 16**
**D-8000 München 86(DE)**

(54) **Respiratory gas flow control apparatus.**

(57) This invention provides an apparatus and method for controlling the flow of gas to a patient. Thus the invention has a sensor which detects when the patient is exhaling and a valve which is operated by the sensor to interrupt the supply of gas. The sensor further senses when the patient inhales, and then operates the valve to resume supply of the gas. A warning means is also provided to detect if the valve remains closed, and to supply a warning signal if that happens.

FIG 1

THE CONTROL OF THE SUPPLY OF GAS TO A
PATIENT

THIS INVENTION relates to a control apparatus for controlling the supply of a gas to a patient for respiratory purposes and to a method of controlling the supply of gas to the patient. It is to be noted that a patient could be a person or animal.

It often occurs that a person, due to some or other defect or illness, cannot inhale sufficient air, and particularly oxygen, to satisfy his needs. To overcome this, it is commonplace to supply the patient pure oxygen gas which then supplements the oxygen he inhales from the air in the atmosphere, the above problem thereby being at least partially overcome. This oxygen gas is, in practice, with free respiration, merely fed from a gas container to the patient, at a suitably required flow rate, via a face pose. As such, it will be appreciated that the oxygen is fed to the person both while inhaling and exhaling, this clearly resulting in oxygen being wasted.

0125424

According to the invention there is provided a control apparatus for controlling the supply of a gas to a patient, which includes

a sensing means for sensing when the patient exhales; and

a valve means responsive to the sensing means for interrupting the flow of gas to the patient when the patient exhales.

Further according to the invention there is provided a method of controlling the supply of gas to a patient which includes sensing when the patient exhales, and interrupting the flow of gas to the patient when the patient exhales.

The sensing means may also sense when the patient inhales, the valve means then being operated to permit flow of gas to the patient.

The valve means may particularly be connected in line with an elongate tubular element defining a flow line through which gas flow is controlled from a supply thereof to a face mask.

The tubular element may be connectable to a gas supply container, which container may form a part of the apparatus of the invention or, alternatively, may be a separate replaceable unit. A flow regulator may be provided to regulate gas flow from the container, permitting gas to flow at a constant rate from the container. The flow regulator may also form a part of the apparatus of the invention or, alternatively, may be provided together with a separate replaceable container.

In one embodiment of the invention, the sensing means may be a mechanical sensor which can sense the movement of the body of a person when inhaling and exhaling. In such a configuration, the sensor may be a device securable with respect to the body of a person, in a region where body movement due to inhaling and exhaling takes place, by means of a strap, any variation in the tension on the strap due to such body movement being sensed by the device. As such, one end or both ends of the strap secured to the device may be engaged with a switching element, secured in series with a control valve comprising the valve means, operation of the control valve being determined by the position of the switching element. As such, with the sensor being strapped around the chest of a person, any increase in tension on the strap when inhaling will displace the switching element into a position in which the control valve is opened to permit gas flow to the person and any relative decrease in tension on the strap will cause the switching element to be displaced into a position in which the control valve is closed to block-off the said flow line.

In order to ensure that it is merely the variation in tension on the strap which determines the displacement of the switching element, a clutch mechanism may be associated with the switching element which is operative so that when tension increases the switching element will be displaced whatever its existing position and when the tension decreases the switching element will be displaced again irrespective of the actual existing position of the switching element. This ensures the sensitivity of the sensor at all times.

The control valve may conveniently be a suitable solenoid valve, the control apparatus including a suitable power source such as a battery to provide the necessary current flow for the operation of the valve. It will be appreciated that the sensor and/or the control valve may be any alternative means which can sense when a person inhales and when a person exhales and which can control the flow of a gas in response thereto respectively. For the above defined configuration, the solenoid valve may be biassed into its open position when the current is broken and displaced into its closed position when its solenoid is activated.

Furthermore, the control apparatus may include a warning means adapted to provide a warning signal against malfunctioning thereof. In particular, the warning means may be adapted to sense when the control valve has not permitted any gas flow for a predetermined period of time, providing a suitable warning signal in response to this occurring. The warning means may also act as a safety means, or alternatively, a separate safety means may be provided, which, when the continued operation of the control valve is sensed as envisaged above, may render the control valve inoperative thus opening the flow line and permitting the continued flow of gas to the patient.

- 6 -

0125424

The invention is now described, by way of an example, with reference to the accompanying diagrammatic drawings, in which

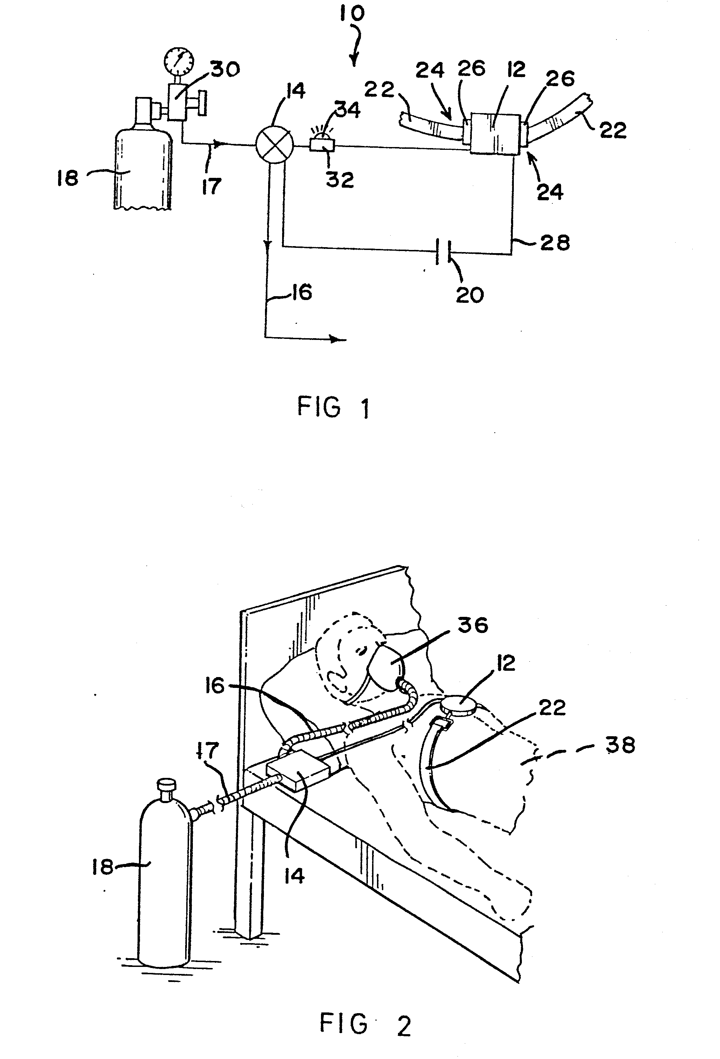

Figure 1 shows schematically a control apparatus for controlling the supply of gas to a person for respiratory purposes, in accordance with the invention; and

Figure 2 illustrates how the apparatus is used.

Referring to the drawings, a control apparatus for controlling the supply of a gas to a person for respiratory purposes, in accordance with the invention, is generally indicated by reference numeral 10. The apparatus 10 generally includes a sensor 12 which is adapted to sense when a person inhales and when a person exhales and a control valve 14 provided to control the flow of a gas through a flow line 16, 17 from a gas container 18.

The control valve 14 is a solenoid valve operable by means of an electric power source in the form of a battery 20, the valve 14 and battery 20 both being connected in series with the sensor 12, which effectively serves as a switch for controlling the operation of the control valve 14 as is described hereinafter. The valve 14 is effectively biassed into an open position and is activated to move into a closed position when its solenoid is activated.

The sensor 12 is essentially a mechanical device which can sense chest expansion and contraction of a person due to breathing. Thus, the sensor 12 is securable about the chest of a person by means of a strap 22 which is tightened around the body of the person and the tension in which will obviously vary the person inhales and exhales. In particular, it will be appreciated that when the person inhales the tension in the strap 22 will increase and when the person exhales the tension in the strap 22 will decrease. The ends 24 of the strap 22 are each connected to switching elements 26 which are displaceable between open positions in which the electric circuit 28 is broken and the control valve 14 is in an open position permitting flow through the tubes 16, 17 and closed positions in which the battery 20 supplies current to the valve 14 causing it to switch into a closed position in which the tubes 16, 17 are blocked off. More particularly, the open position of the switching elements 26 occurs when tension in the straps 22 increases during inhaling by a person and closing of the switching elements 26 occurs when tension in the strap 22 decreases during exhaling.

As such, it will be appreciated that while a person inhales flow of a gas from the container 18 via the tubes16, 17 and control valve 14 is possible whereas when the person exhales such flow is blocked off by means of the control valve 14. To permit operation of the switching elements to be sensitive only to either an increase or a decrease in the tension in the strap 22, irrespective of the actual tension in the strap, one of the switching elements 26 is mounted via a clutch mechanism on a pivotal shaft to be pivotal in a restricted manner and the other is fixed which ensures that the relative angular positions of these elements due to the tension in the straps 22 does not effect their switching operation when variations in tension occur. The applicant believes that this function can be fulfilled in various configurations and the particular configuration described is thus not shown in any specific detail.

In use, in order to supplement the oxygen supply to a person, the flow line 17 is connected to the container 18 containing a supply of pressurised oxygen. The container 18 includes a flow regulator 30 which is then opened to provide a predetermined rate of oxygen release from the container 18 to be fed through the tubes 17. The flow line 16 has at its free end a face mask 36 which can be attached to a person's head. The sensor 12 is next strapped onto the person and when this is rendered operative it will be appreciated that oxygen will be supplied to the person while he inhales and that this oxygen supply will be interrupted when he exhales. A minimum of oxygen is thereby wasted and the continued discomfort of having oxygen blown into the nose and mouth, particularly while exhaling is also greatly reduced. It is believed that considerable savings can result as the effective oxygen supply can be cut off by the control valve for up to 60% of the total time of use during which a person does not usually positively inhale oxygen.

The apparatus 10 may further include a warning means 32 including a warning light 34 which is sensitive to the continued operation of the control valve 14 when blocking off the flow line 16. Accordingly, if the switching elements 26 remain in their closed position indicating exhaling by a person longer than a predetermined period of time, the warning light 34 may automatically switch on indicating that this is happening to the user 38 of the apparatus 10 or any other person. Any other warning signal can clearly also be provided. This will clearly give an indication that oxygen is not being supplied via the tubes 16,17 and the control apparatus 10 can hence be rendered inoperative thus allowing the valve 14 to open and again permit oxygen flow through the flow line 16. Alternatively, or in addition, the warning means 32 may also include a safety means which will in itself open the circuit 28 when the warning signal is emitted, thus permitting the valve 14 to open and hence allow oxygen flow therethrough.

It is anticipated by the applicant that the invention can be performed in various different embodiments still falling within the broad concept of the invention which includes the provision of a sensor for sensing inhaling and exhaling by a person and a control valve to control the supply of gas to the person only when he inhales. For example, more sophisticated sensors are envisaged by the applicant such as electronic sensors or pressure sensors which are specifically adapted for the required purpose. In the same way, alternative valves can be utilised for controlling flow through the flow line 16 to allow a gas such as oxygen to be fed to a person. Still further, the container 18 and the regulator 30 may form a part of the apparatus 10 in which case a single unit can be provided which can be used by a person to supplement his oxygen or any other gas supply during breathing.

Although the use of oxygen has been specifically described the gas may be any other gas.

CLAIMS

1.      A control apparatus for controlling the supply of a gas to a patient the apparatus including

a sensing means for sensing when the patient exhales; and

a valve means responsive to the sensing means for interrupting the flow of gas to the patient when the patient exhales.

2.      An apparatus as claimed in Claim 1, in which the sensing means senses when the patient inhales and the valve means is further responsive to the sensing means for permitting the flow of gas to the patient when the patient inhales.

3.      An apparatus as claimed in Claim 1 in which the sensing means is responsive to movement of a part of the patient's body upon exhalation.

4.      An apparatus as claimed in Claim 3, in which the sensing means includes  an electrical switch means for operating the valve means when exhalation is sensed.

5.      An apparatus as claimed in Claim 4, in which the sensing means includes a suitable strapping means which is connected to the switch means and may be straped around a portion of the body of a patient, such that exhalation causes a decrease in the tension of the strapping means and operation of the switch means.

6.      An apparatus as claimed in Claim 4, in which the valve means comprises a solenoid valve.

7.      An apparatus as claimed in Claim 1, which includes a connecting means for connecting the patient to a source of the gas, the valve means being intermediate inlet and outlet ends of the connecting means.

8.      An apparatus as claimed in Claim 7, in which the connecting means includes a mask connectable to the patient.

9.      An apparatus as claimed in Claim 1 which includes a warning means for supplying a warning signal when the valve means has not permitted flow of gas for a predetermined period of time.

- 13 -    0125424

10.    A method of controlling the supply of gas to a patient which includes sensing when the patient exhales, and interrupting the flow of gas to the patient when the patient exhales.

11.    A method as claimed in Claim 10, which includes sensing when the patient inhales and permitting flow of gas when the patient inhales.

12.    A control apparatus for controlling the supply of gas to a patient, substantially as described in the specification with reference to the accompanying drawings.

13.    A method of controlling the supply of gas to a patient, substantially as herein described and illustrated.

FIG 1

FIG 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A-3 493 703 (J. FINAN) <br><br> * Whole document * | 1-7,10 -13 | A 61 M 16/00 |
| Y | | 9 | |
| | --- | | |
| X | US-A-3 276 462 (J. MATCHETT) <br><br> * Figures; column 1, lines 8-12 * | 1,2,7, 8 | |
| | --- | | |
| Y | FR-A-2 125 449 (DR. FRESENIUS KG) <br> * Figures; page 1, lines 1-15 * | 9 | |
| | ----- | | |

**TECHNICAL FIELDS SEARCHED (Int. Cl. ³)**

A 61 M

The present search report has been drawn up for all claims

| Place of search THE HAGUE | Date of completion of the search 13-06-1984 | Examiner VEREECKE A. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82